# EUROPEAN PATENT SPECIFICATION

(11) **EP 1 438 023 B1**
(45) Date of publication and mention of the grant of the patent: **01.03.2006**
(21) Application number: 02770107.7
(22) Date of filing: 23.10.2002
(51) Int. Cl.: A61K 9/14

(54) **Process for preparing crystalline particles of fluticasone, beclomethasone, salmeterol and salbutamol**
Prozess zur Herstellung kristalliner Partikel von Fluticason, Beclomethason, Salmeterol und Salbutamol
Procédé pour la préparation des particules cristallines de fluticasone, beclomethasone, salmeterol et salbutamol

(30) Priority: 25.10.2001 GB 0125604
(43) Date of publication of application: 21.07.2004
(73) Proprietor: GLAXO GROUP LIMITED, Greenford, Middlesex UB6 ONN (GB)
(72) Inventor: MCLOUGHLIN, Martin, John, Ware, Hertfordshire SG12 0NY (GB)
(74) Representative: Shore, Andrew David
(86) International application number: PCT/GB2002/004820
(87) International publication number: WO 2003/035035

(56) References cited:
- WO-A-00/38811
- WO-A-01/32125
- FR-A- 2 669 635
- US-A- 4 855 436

## Description

The present invention relates to a novel process for isolating crystalline particles of fluticasone, beclomethasone, salmeterol salbutamol or an ester, salt or solvate thereof.

Crystallisation of substances may be achieved via a procedure which involves dissolving the substance of interest in a liquid solvent, followed by mixing with a liquid anti-solvent for said substance, which anti-solvent is generally miscible with the solvent.

US 5314506 (Merck) describes such a process wherein the substance/solvent and anti-solvent are mixed by impinging flows.

WO 00/38811 (Glaxo Group Limited) describes a process for preparing crystalline particles which comprises mixing the solvent and anti-solvent flows in the presence of ultrasound radiation.

WO01/32125 (Glaxo Group Limited) describes a process for preparing crystalline particles which comprises introduction of a stream of substance/solvent tangentially to a stream of anti-solvent in a cylindrical mixing chamber.

A requirement of the above-mentioned solvent/anti-solvent crystallisation approach is that the resultant crystalline particles must be isolated or "harvested" from the suspension in which they are formed. Particle isolation is a crucial stage because any delays in isolation may cause particle size growth if the substance possesses any significant solubility in the supematent liquor (i.e. the solvent/anti-solvent mixture) in which it is suspended. Such particle growth affects the control of the process and is especially undesirable for small (e.g. micron size) particles such as those suitable for inhalation therapy.

A basic method of harvesting the crystalline particles from the liquid in which they are suspended is by filtration e.g. under vacuum or over-pressure.

However many substances have a tendency to form a hard filter cake formed from agglomerates of particles which do not readily disperse. If this occurs the object of producing particles of controlled particle size (especially of small e.g. micron particle size) is not achieved.

We have now invented a new harvesting method which overcomes or subtantially mitigates the above mentioned disadvantage.

Surprisingly, we have found that by mixing the suspension of crystalline particles in the liquid solvent/anti-solvent mixture in which they were formed with a third liquid which is immiscible with said solvent/anti-solvent mixture or a component thereof and thereby forms a second liquid phase, and which is a non-solvent for the substance, and which wets the drug in preference to the solvent/anti-solvent mixture, the substance may be segregated into the second liquid phase. The solvent/anti-solvent mixture may then be separated from the second liquid phase having the substance now suspended in it and the substance may be separated from the second liquid phase e.g. by filtration. Under these conditions we have found that a very light filter cake is generally formed in which the particles are not agglomerated and which particles may be readily dispersed in a subsequent liquid.

Thus, according to a first aspect of the invention there is provided a process for preparing crystalline particles of fluticasone, beclomethasone, salmeterol salbutamol or an ester, salt or solvate thereof which comprises mixing a solution of the substance in a liquid solvent with a liquid anti-solvent for said substance, which liquid anti-solvent is miscible with the liquid solvent, characterised in that the resultant crystalline particles generated are harvested by a process which comprises (i) mixing a first liquid phase formed from the resultant suspension of crystalline substance in solvent/anti-solvent mixture with a third liquid which is immiscible with said solvent/anti-solvent mixture or a component thereof thereby forming a second liquid phase and which is a non-solvent for the substance such that the substance is segregated into the second liquid phase (ii) separating the two phases and (iii) separating the crystalline particles of substance from the second liquid phase.

The third liquid should preferably be immiscible with at least the anti-solvent component of the solvent/anti-solvent mixture.

When the third liquid is miscible with the solvent component of the solvent/antisolvent mixture the solvent will partition between the two liquid phases until equilibrium is achieved. This may result in finite solubility of the substance in the second liquid phase and also reduce the density difference between the two layers, therefore, reducing separation efficiency. Further addition of anti-solvent will however effectively reduce the concentration of the undesired solvent component in the second liquid phase, minimising the solubility of the substance and maximising the density difference between the two liquid layers. Thus step (i) may optionally include the step of adding further anti-solvent to the liquids and mixing.

As a consequence of the immiscibility between the 2 liquid phases, mixing in step (i) will preferably be strong enough to ensure that complete wetting of the substance by the third liquid has occurred. Use of a rotary or magnetic stirrer may be suitable for this purpose.

Preferably the volume of third liquid added to the solvent/anti-solvent mixture is significantly smaller than that of the solvent/anti-solvent mixture (e.g. less than 25% of the volume) such that the suspension of substance in the second liquid phase is more concentrated than that formerly in the first liquid phase. This concentration effect results in processing advantages.

The third liquid should be a sufficiently poor solvent for the substance such that no appreciable dissolution of the substance occurs in the second liquid phase such that the particles produced from the crystallisation are stable in size and do not grow at any appreciable rate. In particular it should be a sufficiently poor solvent for the substance such that bridging between particles in any wet filter cake formed prior to drying does not occur to any significant extent.

Preferably the third liquid together with any solvent that have partitioned into the second liquid phase is a poorer solvent for the substance than the solvent/anti-solvent mixture. This results in lesser likelihood of bridging of particles during drying.

Once the substance has been completely wetted, upon standing the liquid phases will separate with the substance preferentially segregated into the second liquid phase. The forces associating the substance with the third liquid may be quite strong, and may be capable of overcoming the gravitational force when the substance is more dense than either liquid phase.

In step (ii) the liquid phases may be separated by decanting the less dense phase off the more dense phase. Alternatively the more dense phase may be funnelled or gravity fed off the less dense phase.

In step (iii) the second liquid phase may be removed from the suspension of substance in second liquid phase to yield particulate substance eg by filtration of centrifugation. As noted above, such filter cakes tend to be light and permit ready re-dispersion in a subsequent liquid. Alternatively in a preferred method of operation the second liquid phase may be removed by evaporation. This method is, however, less suitable if the concentration of any solvent dissolved in the third liquid is appreciable and the solvent is less volatile than the third liquid. Evaporation may require heating depending on the vapour pressure of the third liquid or second liquid phase.

Alternatively the second liquid phase may be extracted into a stream of gaseous phase eg supercritical carbon dioxide under pressurised conditions provided that the substance is not soluble in the gaseous phase. Under these conditions dry particles may be obtained following depressurisation of the apparatus.

In a further alternative embodiment, the third liquid is a gas at room temperature and pressure and is used in the process under pressure as a liquid. In step (iii) separation may be achieved by releasing the pressure to permit vaporisation of the second liquid phase.

Preferably, the third liquid will have a low boiling point such that its separation from the substance may be achieved by exposure to atmospheric pressure or moderate vacuum and ambient or moderately elevated temperature (e.g. up to 40 °C).

Preferably the crystalline particles are produced by a continuous process which comprises mixing a solution of the substance in a liquid solvent with liquid anti-solvent for said substance such that crystalline particles are generated.

For example, a process for preparing crystalline particles of substance comprises mixing in a continuous flow cell in the presence of ultrasonic radiation a flowing solution of the substance in a liquid solvent with a flowing liquid anti-solvent for said substance, which is miscible with the liquid solvent, and collecting the resultant crystalline particles generated.

A feature of the process is that in a steady state the concentration of dissolved substance in the mixing chamber of the flow cell remains approximately constant since the precipitating substance is replaced by the inflow of further solution. This allows the process to be run continuously and reproducibly.

An apparatus for preparing crystalline particles of a substance in this manner comprises:
(i) a first reservoir of said substance dissolved in a liquid solvent;
(ii) a second reservoir of liquid anti-solvent for said substance;
(iii) a mixing chamber having first and second inlet ports and an outlet port;
(iv) means for delivering the contents of the first and second reservoirs to the mixing chamber via the first and second inlet ports respectively at independent controlled flow rate;
(v) a source of ultrasonic radiation located in the vicinity of the first inlet; and
(vi) means for collecting crystalline particles suspended in the liquid discharged from the mixing chamber at the outlet port.

Alternatively a process for preparing crystalline particles of substance comprises (i) admitting a stream of solution of the substance in a liquid solvent and a stream of liquid anti-solvent for said substance which is miscible with the liquid solvent tangentially into a cylindrical mixing chamber having an axial outlet port such that said streams are thereby intimately mixed through formation of a vortex and precipitation of crystalline particles of the substance is thereby caused; and (ii) collecting the resultant crystalline particles suspended in the stream of liquid discharged from the outlet port of the mixing chamber.

Crystalline particles may also be prepared by batch processes. Typically the solvent and substance will be mixed in a vessel and heated to assist solubilisation of the substance, and then the anti-solvent added to induce crystallisation.

The process of the present invention is particularly suitable for preparing particles of substances which are pharmaceutical or carrier substances suitable for inhalation therapy.

Substances suitable for inhalation therapy include substances applied topically to the lung and nose.

Examples of pharmaceutical substances suitable for inhalation therapy include; anti-inflammatories, e.g., beclomethasone (e.g. as the dipropionate ester); fluticasone (e.g. as the propionate), bronchodilators, e.g., salmeterol (e.g. as the xinafoate), or salbutamol.

Pharmaceutical substances of particular interest include fluticasone, beclomethasone, salmeterol, salbutamol or an ester, salt or solvate thereof. The substance of most interest is salmeterol xinafoate (including the racemate or the purified r- or s- enantiomers). Fluticasone propionate is also of particular interest.

Examples of carrier substances include lactose.

The solvent and anti-solvent liquids will be selected so as to be appropriate for the substance. They should be readily miscible in the proportions employed. Suitable combinations of solvent/antisolvent include acetone/water, ethanol/IPA, methanol/IPA, methanol/water and reciprocal pairs. Methanol/IPE is also a suitable pairing. As noted above the third liquid will be selected so as to be immiscible with the solvent/anti-solvent mixture or a component thereof and so as to be a non-solvent for the substance.

The solvent and anti-solvent may each be mixtures of liquids if desired or necessary.

For generation of small particles by the process according to the invention, it is preferred that the difference between the dissolution properties of the solvent and anti-solvent be as great as possible. For reasons of industrial efficiency (particularly in order to reduce the throughput volumes of liquid) it is preferred to use concentrations of substance in solvent which are as high as possible. Nevertheless the solutions must be stable and not prone to crystallisation before discharge into the continuous flow cell. With this end in mind, it may be preferred to use the solution of the substance in the solvent at elevated temperature. It may also be preferable to cool the anti-solvent.

In order to prevent premature precipitation of the dissolved substance in the lines it will generally be desired to prime the apparatus by first pumping it with solvent. It may be preferred to prime the apparatus by pumping it with heated solvent, particularly when the dissolved substance is close to its solubility limit.

When the substance is fluticasone propionate we prefer the solvent to be acetone and the anti-solvent to be water. Preferably the third liquid is a lower alkane which is a liquid at ambient temperature and pressure e.g. hexane, pentane, heptane or iso-octane, especially n-hexane. A lower alkane which is liquified under pressure e.g. butane may also be employed. Ethers may also be suitable.

When the substance is salmeterol xinafoate we prefer the solvent to be methanol or acetone (more preferably methanol) and the anti-solvent to be water. Preferably the third liquid is a lower alkane which is a liquid at ambient temperature and pressure eg hexane, pentane, heptane or iso-octane, especially n-hexane. A lower alkane which is liquified under pressure e.g. butane may also be employed.

When the substance is salbutamol sulphate we prefer the solvent to be water and the anti-solvent to be IMS.
When the substance is beclomethasone dipropionate we prefer the solvent to be IMS and the anti-solvent to be water.

The preferred third liquid is a lower alkane which is a liquid at ambient temperature and pressure eg hexane, pentane, heptane or iso-octane, especially n-hexane. The preferred anti-solvent is immiscible with such a third liquid and the preferred solvent, which will be selected accordingly, will preferably be immiscible with such a third liquid.

We have found that the method according to the invention is suitable for producing populations of mixtures when the substance is a mixture of substances. When the substance is a mixture the method has particular advantages since it is capable of producing mixtures of crystalline particles of very high homogeneity without the need for any blending step. When the substance is a mixture the solvent and anti-solvent will have to be appropriate for all components of the mixture. Differential solubilities in the recrystalline mixture tend to result in the output proportions of the mixture differing from the initial proportions in solution in the solvent and so appropriate adjustment of the input proportions to achieve the desired output proportions may be necessary.

The method according to the invention is particularly suitable for producing mixtures of crystalline particles of salmeterol and fluticasone or salts and esters thereof e.g. salmeterol xinafoate and fluticasone propionate. The preferred solvent is acetone. The preferred anti-solvent is water. The preferred third liquid is a lower alkane which is a liquid at ambient temperature and pressure eg hexane, pentane, heptane or iso-octane, especially n-hexane Recrystallisation from acetone using water as anti-solvent tends to cause an increase in the ratio of salmeterol xinafoate to fluticasone propionate relative to their proportion in solution in acetone. The method is also expected to be suitable for producing mixtures of crystalline particles of formoterol and budesonide or salts and esters thereof e.g. formoterol fumarate and budesonide.

As a further aspect of the invention we provide a population of particles obtainable by a process according to the invention.

Particles of pharmaceutical or carrier substances may be obtained which are suitable for use in a pharmaceutical composition for inhalation therapy, such as dry powder composition (whether containing pure drug, or drug mixed with a carrier such as lactose) or a pressurised liquid formulation (e.g. a formulation comprising a hydrofluoroalkane (HFA) propellant such as HFA134a or HFA227 or a mixture thereof).

Pressurised liquid formulations suitable for metered-dose inhalers will be retained in canisters, typically aluminium canisters (which may be plastics lined) which are provided with a metering valve of appropriate metering volume.

It will be appreciated that references to inhalation therapy also extend to administration of pharmaceutical compositions via the nasal route. Formulations suitable for nasal delivery include pressurised (e.g. HFA containing) formulations and non pressurised (e.g. aqueous) formulations which may be metered by the delivery device adapted for administration to the nose.

We also provide a pharmaceutical composition comprising a population of particles prepared according to the invention.

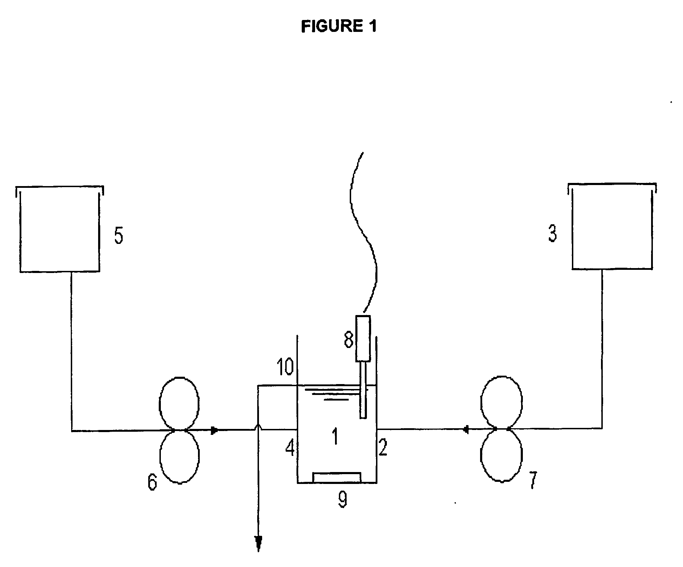

Apparatus suitable for use in the present invention is illustrated by reference to Figure 1 in which mixing chamber 1 is provided with first inlet port 2 connected to first reservoir 3 containing substance dissolved in solvent and second inlet port 4 connected to second reservoir 5 containing anti-solvent. Pumps 6 and 7 deliver liquid from reservoirs 3 and 5 to mixing chamber 1 at a controlled rate. An ultrasound probe 8 is located in the vicinity of, and just above, inlet port 2. When pumps 6 and 7 are in operation, liquids from reservoirs 3 and 5 are delivered to mixing chamber 1 and are mixed with the aid of magnetic stirrer 9. Liquid containing the particles of substance thus generated flows out of the mixing chamber via exit port 10. Details not shown: this suspension may be mixed with the third liquid, vigorously mixed, and the second phase containing third liquid and substance decanted or funnelled off. The substance may be harvested from the liquid phase by fltration followed by drying.

### Brief description of the drawings.

Figure 1 shows example apparatus suitable for use according to the invention.

The present invention is further illustrated by the following Examples:

### Example 1: Isolation of fluticasone propionate

Fluticasone propionate is not wetted by water nor is it soluble in water (i.e. it is hydrophobic in nature). However, fluticasone propionate is readily soluble in acetone. Therefore, fluticasone propionate was crystallised in accordance with the process described in WO 00/38811, wherein 30g of fluticasone propionate was yielded from a 1:4 w/w ratio of solvent :anti-solvent (acetone:water). The resultant liquor composition comprised >60% w/w water and as a consequence fluticasone propionate was insoluble in the resultant composition.
The resultant liquor composition was added to a 5L duran flask containing between 50 and 150m1 hexane as third liquid and 2L of distilled water (which was added to reduce the acetone concentration in the alkane layer by partitioning and increasing the density difference between the liquid layers, resulting in improved separation efficiency). The addition of the slurry immediately resulted in the formation of two liquid layers, the uppermost being hexane/acetone and the lower being water/acetone.

The duran flask was then vigorously shaken to break the interface between the layers causing full wetting of the fluticasone propionate by the alkane. The duran flask was then left to stand wherein after 5 minutes it was observed that most of the fluticasone propionate had segregated into the alkane layer. Complete segregation was achieved within 12 minutes and the upper layer containing substance was decanted off. The substance was separated from the liquid phase and dried by filtration under vacuum overnight. The resultant particles were light, low density and readily dispersed.

### Example 2: Isolation of salmeterol xinafoate

20 ml of a solution of 40mg/ml salmeterol xinafoate in methanol was precipitated by addition to 180ml of de-mineralised water at room temperature, using a syringe. The salmeterol xinafoate was rapidly precipitated to form a milky, opaque suspension of salmeterol xinafoate in water-methanol. The suspension showed little tendency to settle, indicating relatively fine particles. Subsequently 20 ml of hexane was added to the suspension which was then shaken vigorously. On settling, the salmeterol xinafoate flocculated and extensively partitioned into the hexane layer. Further vigorous shaking and subsequent standing resulted in almost all of the salmeterol xinafoate partitioning into the hexane layer. The hexane layer was decanted from the methanol-water layer and vacuum filtered until dry.

## Claims

1. A process for preparing crystalline particles of substance which is a pharmaceutical selected from ftuticasone, beclornothasons, salmeterol, salbutamol or an ester, salt or solvate thereof which comprises mixing a solution of the substance In a liquid solvent with a liquid anti-solvent for said substance, which liquid anti-solvent is miscible with the liquid solvent, **characterised in that** the resultant crystalline particles generated are harvested by a process which comprises:
(i) mixing a first liquid phase formed from the resultant suspension of crystalline substance in solvent/anti-solvent mixture with a third liquid which is immiscible with said solvent/anti-solvent mixture or a component thereof thereby forming a second liquid phase and which is a non-solvent for the substance such that the substance is segregated into the second liquid phase;
(ii) separating the two phases: and
(iii) separating the crystalline particles of substance from the second liquid phase.

2. A process according to claim 1 wherein the mixing of a solution of the substance in a liquid solvent with a liquid anti-solvent for said substance is carried out in a continuous flow cell in the presence of ultrasonic radiation.

3. A process according to claim 1 or 2 in which the third liquid is immiscible with at least the anti-solvent component of the solvent/anti-solvent mixture.

4. A process according to any of the preceding claims in which the volume of the third liquid added to the solvent/anti-solvent mixture is less than 25% of the total volume.

5. A process according to any one of the preceding claims in which the third liquid is a lower alkane which is a liquid at ambient temperature and pressure.

6. A process according to claim 5 in which the lower alkane is n-hexane.

7. A process according to any of the preceding claims in which in step (ii) the liquid phases are separated by decanting the less dense phase off the more dense phase.

8. A process according to any one of the preceding claims in which in step (iii) the crystalline particles of substance are separated from the second liquid phase by evaporation.

9. A process according to any one of claim 1 - 8 in which in step (iii) the crystalline particles of substance are separated from the second liquid phase by extraction into a supercritical fluid.

10. A process according to claim 1 wherein the substance is fluticasone propionate.

11. A process according to claim 1 wherein the substance is salmeterol xinafoate.

12. A process according to any one of claims 1 - 9 wherein the substance is a mixture.

13. A process according to claim 12 wherein the substance is a mixture of fluticasone propionate and salmeterol xinafoate.

14. A process according to claim 10 or 13 wherein the solvent is acetone and the anti-solvent is water.

15. A process according to claim 11 wherein the solvent is methanol and the anti-solvent is water.

## Patentansprüche

1. Verfahren zur Herstellung kristalliner Partikel aus Substanz, die ein Pharmazeutikum ist, das aus Fluticason, Beclomethason, Salmeterol, Salbutamol oder einem Ester, Salz oder Solvat davon ausgewählt ist, welches das Vermischen einer Lösung der Substanz in einem flüssigen Lösungsmittel mit einem flüssigen Antilösungsmittel für die Substanz umfaßt, wobei das flüssige Antilösungsmittel mit dem flüssigen Lösungsmittel mischbar ist, **dadurch gekennzeichnet, daß** die resultierenden erzeugten kristallinen Partikel durch ein Verfahren geerntet werden, das die folgenden Schritte umfaßt:
(i) Vermischen einer ersten flüssigen Phase, die aus der resultierenden Suspension von kristalliner Substanz in Lösungsmittel/Antilösungsmittel-Mischung gebildet ist, mit einer dritten Flüssigkeit, die mit der Lösungsmittel/Antilösungsmittel-Mischung oder einer Komponente davon unmischbar ist, wodurch eine zweite flüssige.Phase gebildet wird, und die ein Nichtlösungsmittel für die Substanz ist, so daß die Substanz in die zweite flüssige Phase abgesondert wird;
(ii) Trennen der zwei Phasen; und
(iii) Abtrennen der kristallinen Partikel aus Substanz von der zweiten flüssigen Phase.

2. Verfahren gemäß Anspruch 1, worin das Vermischen einer Lösung der Substanz in einem flüssigen Lösungsmittel mit einem flüssigen Antilösungsmittel für die Substanz in einer kontinuierlichen Durchflußzelle in Gegenwart von Ultraschall durchgeführt wird.

3. Verfahren gemäß Anspruch 1 oder 2, worin die dritte Flüssigkeit mit zumindest der Antilösungsmittelkomponente der Lösungsmittel/Antilösungsmittel-Mischung unmischbar ist.

4. Verfahren gemäß einem der vorhergehenden Ansprüche, worin das Volumen der zu der Lösungsmittel/Antilösungsmittel-Mischung hinzugegebenen dritten Flüssigkeit weniger als 25 % des Gesamtvolumens ist.

5. Verfahren gemäß einem der vorhergehenden Ansprüche, worin die dritte Flüssigkeit ein niederes Alkan ist, das bei Umgebungstemperatur und -druck flüssig ist.

6. Verfahren gemäß Anspruch 5, worin das niedere Alkan n-Hexan ist.

7. Verfahren gemäß einem der vorhergehenden Ansprüche, worin in Schritt (ii) die flüssigen Phasen durch Abdekantieren der weniger dichten Phase von der dichteren Phase getrennt werden.

8. Verfahren gemäß einem der vorhergehenden Ansprüche, worin in Schritt (iii) die kristallinen Partikel aus Substanz von der zweiten flüssigen Phase durch Verdampfung abgetrennt werden.

9. Verfahren gemäß einem der Ansprüche 1 bis 8, worin in Schritt (iii) die kristallinen Partikel aus Substanz von der zweiten flüssigen Phase durch Extraktion in eine überkritische Flüssigkeit abgetrennt werden.

10. Verfahren gemäß Anspruch 1, worin die Substanz Fluticasonpropionat ist.

11. Verfahren gemäß Anspruch 1, worin die Substanz Salmeterolxinafoat ist.

12. Verfahren gemäß einem der Ansprüche 1 bis 9, worin die Substanz eine Mischung ist.

13. Verfahren gemäß Anspruch 12, worin die Substanz eine Mischung aus Fluticasonpropionat und Salmeterolxinafoat ist.

14. Verfahren gemäß Anspruch 10 oder 13, worin das Lösungsmittel Aceton ist und das Antilösungsmittel Wasser ist.

15. Verfahren gemäß Anspruch 11, worin das Lösungsmittel. Methanol ist und das Antilösungsmittel Wasser ist.

## Revendications

1. Procédé pour la préparation de particules cristallines de substance qui est un produit pharmaceutique choisi parmi la fluticasone, la béclométhasone, le salmétérol, le salbutamol ou un ester, sel ou solvate de ceux-ci, qui comprend le mélange d'une solution de la substance dans un solvant liquide avec un liquide anti-solvant pour ladite substance, lequel anti-solvant liquide est miscible avec le solvant liquide, **caractérisé en ce que** les particules cristallines résultantes générées sont récoltées par un procédé qui comprend :
(i) le mélange d'une première phase liquide formée à partir de la suspension résultante de substance cristalline dans un mélange solvant/anti-solvant avec un troisième liquide qui est immiscible avec ledit mélange solvant/anti-solvant ou un composant de celui-ci, de façon à former ainsi une seconde phase liquide et qui est un non solvant pour la substance de telle sorte que la substance est séparée dans la seconde phase liquide ;
(ii) la séparation des deux phases ; et
(iii) la séparation des particules cristallines de substance à partir de la seconde phase liquide.

2. Procédé selon la revendication 1, dans lequel le mélange d'une solution de la substance dans un solvant liquide avec un anti-solvant liquide pour ladite substance est réalisé dans une cellule à écoulement continu en présence de radiation ultrasonique.

3. Procédé selon la revendication 1 ou 2, dans lequel le troisième liquide est immiscible avec au moins le composant anti-solvant du mélange solvant/anti-solvant.

4. Procédé selon l'une quelconque des revendications précédentes, dans lequel le volume du troisième liquide ajouté au mélange solvant/anti-solvant est inférieur à 25% du volume total.

5. Procédé selon l'une quelconque des revendications précédentes, dans lequel le troisième liquide est un alcane inférieur qui est un liquide aux température et pression ambiantes.

6. Procédé selon la revendication 5, dans lequel l'alcane inférieur est le n-hexane.

7. Procédé selon l'une quelconque des revendications précédentes, dans lequel dans l'étape (ii), les phases liquides sont séparées par décantation de la phase moins dense à partir de la phase la plus dense.

8. Procédé selon l'une quelconque des revendications précédentes, dans lequel dans l'étape (iii), les particules cristallines de substance sont séparées de la seconde phase liquide par évaporation.

9. Procédé selon l'une quelconque des revendications 1 à 8, dans lequel dans l'étape (iii), les particules cristallines de substance sont séparées de la seconde phase liquide par extraction dans un fluide supercritique.

10. Procédé selon la revendication 1, dans lequel la substance est le propionate de fluticasone.

11. Procédé selon la revendication 1, dans lequel la substance est le xinafoate de salmétérol.

12. Procédé selon l'une quelconque des revendications 1 à 9, dans lequel la substance est un mélange.

13. Procédé selon la revendication 12, dans lequel la substance est un mélange de propionate de fluticasone et de xinafoate de salmétérol.

14. Procédé selon la revendication 10 ou 13, dans lequel le solvant est l'acétone et l'anti-solvant est l'eau.

15. Procédé selon la revendication 11, dans lequel le solvant est le méthanol et l'anti-solvant est l'eau.
